# FASCICULE DE BREVET EUROPEEN

(11) **EP 3 277 298 B1**
(45) Date de publication et mention de la délivrance du brevet: **21.09.2022**
(21) Numéro de dépôt: 16714856.8
(22) Date de dépôt: 04.04.2016
(51) Int. Cl.: A61K 35/747, A61F 2/00, A61L 31/16, A61K 9/00, A61M 31/00

(54) **REACTEUR INTESTINAL IMPLANTABLE**
IMPLANTIERBARER INTESTINALER REAKTOR
IMPLANTABLE INTESTINAL REACTOR

(30) Priorité: 03.04.2015 FR 1552927
(43) Date de publication de la demande: 07.02.2018
(73) Titulaire: UNIVERSITE GRENOBLE ALPES, 38400 Saint Martin d'Hères (FR); Centre Hospitalier Universitaire Grenoble, 38043 La Tronche (FR)
(72) Inventeur: CINQUIN, Philippe, 38330 Saint Nazaires Les Eymes (FR); SCHNEIDER, Dominique, 38920 Crolles (FR); MAURIN, Max, 38240 Meylan (FR); MARTIN, Donald, 38610 Gieres (FR); EL ICHI RIBAULT, Sarra, 69100 VILLEURBANNE (FR); ZEBDA, Abdelkader, 38100 Grenoble (FR); ALCARAZ, Jean-Pierre, 38530 Pontcharra (FR); RECHE, Fabian, 38190 Sainte Agnes (FR); TUVIGNON, Patrick, 81000 Albi (FR); THELU, Jacques, 38920 Crolles (FR); LE GOUELLEC, Audrey, 38410 Uriage (FR); TOUSSAINT, Bertrand, 38120 Saint Egreve (FR)
(74) Mandataire: Hautier IP - MC/EP
(86) Numéro de dépôt international: PCT/EP2016/057304
(87) Numéro de publication internationale: WO 2016/156612

(56) Documents cités:
- WO-A1-2008/115411
- WO-A2-2007/092390
- US-A1- 2002 103 522
- US-A1- 2004 213 825
- US-A1- 2004 249 362
- US-A1- 2009 035 351
- US-A1- 2010 063 518

## Description

La présente invention concerne un dispositif implantable dans la cavité intestinale, permettant de conduire des réactions de transformation ou de synthèse au sein de l'intestin, et son utilisation pour traiter une pathologie, corriger une dérégulation ou encore prévenir l'apparition d'une pathologie ou d'une dérégulation.

Le glucose est l'exemple typique de molécule dont on peut souhaiter réduire l'absorption intestinale, par exemple, dans le cas de surcharges pondérales ou de diabète de type II ou même de type I.

De nombreuses propositions ont été faites pour permettre à des enzymes ou à des micro-organismes d'atteindre l'intestin sans être dégradés par le passage gastrique. On peut citer par exemple le Lactaid^{®}, un complément alimentaire à base de galactosidase, notamment alpha-galactosidase et beta-galactosidase, destiné à traiter les intolérances au lactose liées à un déficit en cette enzyme. On peut citer de manière plus générale les divers suppléments alimentaires contenant des bactéries, par exemple la bactérie *bidifus.* L'efficacité de ces approches est cependant largement diminuée par le fait que ces produits sont altérés au niveau du grêle par un environnement agressif, en particulier pour des protéines, et par le fait que les composants actifs ne restent pas longtemps dans le duodénum et le jéjunum, zones où les concentrations en glucides, et plus généralement en nutriments divers, sont maximales.

La présente invention a donc pour objectif de proposer un dispositif permettant de rendre des produits actifs biodisponibles au niveau intestinal, qui résolve les problèmes de l'art antérieur.

Un autre objectif de l'invention est de proposer un tel dispositif qui permette une biodisponibilité sur une durée maîtrisable, plus ou moins longue.

Un autre objectif encore de l'invention est de proposer un tel dispositif qui permette de rendre biodisponible une grande variété de produits actifs, allant de la molécule type enzyme et son cofacteur éventuel, à un micro-organisme.

L'invention a donc pour objet un dispositif implantable dans la cavité intestinale telle que défini par la revendication 1. Ce dispositif comprend un réacteur positionnable dans la cavité intestinale. Par implantable, on entend que le dispositif et son réacteur sont maintenus en place dans la cavité intestinale. Positionnable signifie que le réacteur est maintenu à l'endroit où il est initialement positionné. Le réacteur se maintient dans cette position pour la durée d'utilisation, qui peut atteindre plusieurs jours, semaines, mois ou années. Le réacteur peut avantageusement être implanté par voie orale, puis retiré par la même voie, par exemple par endoscopie. Il est maintenu en place grâce à un élément de maintien ou de fixation, comprenant une pièce d'ancrage dans la paroi intestinale ou gastrique ou un stent gastrique ou intestinal, comme cela sera détaillé plus loin.

Le réacteur comprend un matériau biocompatible, de préférence un matériau polymère biocompatible, qui est apte à immobiliser ou contenir un élément ou produit actif. Ce matériau peut être sous la forme d'une membrane, d'une feuille, d'une masse de matériau ou d'un revêtement. Par produit actif, on entend un produit susceptible de générer une réaction chimique ou biologique avec une ou des molécules présentes dans l'intestin ou de produire une ou des molécules potentiellement intéressantes pour l'objet visé par l'invention. Nous détaillerons plus loin des types de produits actifs et leurs applications, mais la notion de produit actif inclus aussi bien les molécules chimiques que les microorganismes vivants.

Par définition, un réacteur peut être d'une part un élément qui renferme un produit actif (placé au préalable dans cet élément) tout en lui permettant d'interagir avec le milieu intestinal soit en libérant au moins un produit actif soit en réagissant avec un ou des éléments contenus dans le milieu intestinal entrant en contact avec le contenu du réacteur. Un réacteur peut être d'autre part un support à un produit actif qui, attaché au support, va interagir avec le milieu intestinal soit en libérant au moins un produit actif soit en réagissant avec un ou des éléments contenus dans le milieu intestinal. Le réacteur est donc défini comme renfermant ou supportant un produit actif, ce qui exclut par exemple un tube ouvert dans lequel s'écoule un produit liquide.

Le réacteur peut comprendre une membrane, une feuille, un matériau ou masse de matériau, ou un revêtement ayant une perméabilité appropriée. Il peut s'agir, mais pas exclusivement, de matière semi-perméable. Le matériau ou masse de matériau destinée à servir de support au produit ou élément actif peut prendre toute forme, par exemple ruban, tube, structure comprenant plus de deux faces, etc.

Dans un premier mode de réalisation, le réacteur peut être un dispositif en matériau biocompatible délimitant, au moins en partie avec sa membrane semi-perméable ou poreuse, un volume intérieur fermé, dans lequel le produit actif peut être placé. Le dispositif comporte alors au moins une surface extérieure délimitée par une membrane semi-perméable ou poreuse réalisée en matériau biocompatible, de préférence en matériau polymère biocompatible. Le dispositif peut comporter une structure intérieure, telle qu'un cadre à rigidité adaptée, donnant sa forme au réacteur. Il peut aussi ne pas comporter de telle structure et être entièrement souple, par exemple un tube ou sac allongé fermé.

Le réacteur a de préférence une forme allongée. Sa longueur peut être notamment comprise entre environ 1 cm et environ 100 cm, en particulier entre environ 1 cm et environ 50 cm, notamment entre environ 1 cm et environ 20 cm, de préférence entre environ 2 cm et environ 10 cm. Sa plus grande largeur ou son diamètre peut être notamment compris entre environ 0,5 cm et environ 3 cm, de préférence entre environ 1 cm et environ 2 cm. L'épaisseur du matériau ou de la membrane peut être comprise entre environ 0,5 mm et environ 2 mm par exemple de l'ordre de 1 mm. Le volume intérieur du réacteur peut notamment être compris entre environ 1 mL et environ 180 mL, de préférence entre environ 2 mL et environ 50 mL.

Le réacteur peut avoir la forme d'un sac allongé ou d'un tube cylindrique, semi-cylindrique ou sensiblement aplati. Il peut également avoir la forme d'un réseau de tubules, voire de fibres. Sa structure est avantageusement souple.

De préférence, la totalité de la surface ou plus d'environ 60, 70, 80 ou 90 % de celle-ci est constituée par la membrane semi-perméable ou poreuse.

Le réacteur peut comporter plusieurs compartiments, par exemple au moins 2. Notamment, le réacteur peut comprendre à l'intérieur du volume délimité par la première membrane, un compartiment dénommé ici deuxième compartiment, délimité par une deuxième membrane semi-perméable ou poreuse, dont les propriétés, notamment la porosité sont différentes de la précédente.

Le réacteur peut être fixé à un cordon. Le cordon est d'une section et d'un matériau qui ne blesse pas le système digestif. Sa section peut être par exemple une section ronde, semi-ronde ou plate. Le cordon peut être continu (mono-brin) ou être un assemblage de fils ou autres unités élémentaires, assemblés pour former le cordon, par exemple sous la forme d'une tresse ou d'une structure textile. Le cordon peut être en matériau organique ou inorganique biocompatible. Il peut s'agir d'un matériau à base de polymère synthétique (par exemple en polyéthylène, e.g. Dacron@) ou à base de métal (par exemple alliage nickel-titane). Il présente une résistance vis-à-vis de l'acidité et des enzymes présentes, ainsi qu'une résistance à la rupture suffisante pour résister aux forces appliquées sur le réacteur par le transit intestinal.

La fixation du réacteur au cordon peut être réalisée de toute manière connue, par exemple par collage, soudure, couture, à l'aide de nœuds, ou encore par le fait que le cordon est d'un seul tenant avec le réacteur. Dans un mode de réalisation, le cordon est relié au réacteur par l'intermédiaire d'un émerillon ou similaire, permettant la rotation de l'un par rapport à l'autre.

Dans un mode de réalisation, le cordon est entouré par des segments cylindriques dont le diamètre intérieur est supérieur au diamètre du cordon de manière que ces segments, dits autogyres, puissent tourner autour du cordon et éviter à celui-ci de se vriller sous l'action des contraintes mécaniques s'exerçant sur le dispositif. L'emploi de segments permet de conserver la souplesse à l'ensemble. Par exemple, des segments d'environ 0,5 à environ 5 cm, de préférence d'environ 1 à environ 4 cm, e.g. d'environ 3 cm, peuvent être utilisés.

A son extrémité opposée à son point d'attache au réacteur, le cordon peut être relié à, ou comporter d'un seul tenant, une pièce d'ancrage. Ce que l'on appelle pièce d'ancrage est un dispositif ou une partie d'un dispositif d'ancrage dans ou sur une paroi tissulaire. De tels dispositifs seront détaillés plus loin. Le cordon a une longueur qui permet, en tenant compte du point d'attache tissulaire, de positionner le réacteur à l'endroit voulu dans l'intestin, notamment dans le duodénum, de préférence en aval de l'abouchement du canal biliaire. Le cordon ou la partie cordon pourra notamment présenter une longueur comprise entre environ 10 cm et environ 50 cm, de préférence entre environ 10 cm et environ 40 cm, plus particulièrement entre environ 15 cm et environ 30 cm. La longueur hors tout réacteur plus cordon peut donc être comprise entre environ 11 cm et environ 150 cm, en particulier environ 11 cm et environ 60 cm.

Le produit actif peut être présent dans le volume intérieur du réacteur sous forme libre ou associée à un support ou une matrice. De préférence, le réacteur contient un matériau de remplissage ou matrice, qui peut être particulaire, granulaire ou sous la forme d'une masse ou bloc unitaire, éventuellement poreuse. Le produit actif peut être simplement mélangé avec ledit matériau, notamment particulaire ou granulaire, ou encore adsorbé ou retenu par des liaisons faibles à sa surface ou associé d'une autre manière connue en tant que telle. Le matériau, notamment lorsqu'il sous forme de masse, peut être poreux ou encore stable ou dissoluble dans les conditions régnant dans l'intestin. A titre d'exemple de matériau, on peut citer le chitosane, l'alginate de baryum, un hydrogel de cellulose, d'agar ou de PVA (Poly-(Alcool Vinylique)).

Dans un deuxième mode de réalisation, le réacteur présente au moins deux faces libres (c'est-à-dire pouvant entrer en contact avec le milieu dans lequel le réacteur est plongé, à savoir le liquide intestinal). Suivant une première modalité, ce réacteur a une géométrie aplatie, il peut avoir la forme d'un ruban ou d'une structure ayant plus de deux faces. Suivant une deuxième modalité, le réacteur est un tube de section quelconque, par exemple ronde, semi-ronde, parallélépipédique, les deux faces susdites étant donc formées par la face extérieure et par la face intérieure.

Des micro-organismes peuvent être immobilisés, un biofilm peut être formé et/ou des molécules actives peuvent être immobilisées sur au moins l'une des deux faces de ce réacteur.

Dans la première modalité, ce réacteur peut avoir la forme d'un ruban lisse, éventuellement sous forme d'hélice. Il peut aussi avoir une structure dans laquelle le réacteur présente des irrégularités locales permettant d'augmenter la superficie d'échange par rapport à la surface apparente. Il peut encore avoir une structure complexe avec des sections en L, T, étoile à 4, 5, 6 branches ou plus, etc., ce qui permet de multiplier les surfaces sur lesquelles le produit actif peut être immobilisé.

Le réacteur peut être fixé à un cordon, du type défini ci-dessus. La fixation du réacteur au cordon peut être réalisée de toute manière connue, par exemple par collage, soudure, couture, à l'aide de nœuds. Un émerillon ou similaire peut être utilisé. Le cordon peut être équipé des segments autogyres mentionnés plus haut. Le cordon a une longueur qui permet, en tenant compte du point d'attache tissulaire, de positionner le réacteur à l'endroit voulu dans l'intestin, notamment dans le duodénum, de préférence en aval de l'abouchement du canal biliaire. Le cordon ou la partie cordon pourra notamment présenter une longueur permettant de positionner correctement le réacteur compte tenu de la longueur de ce dernier. La longueur hors tout réacteur plus cordon peut être notamment comprise entre environ 1 cm et environ 150 cm, en particulier entre environ 1 cm et environ 50 cm, de préférence entre environ 10 cm et environ 40 cm, plus particulièrement entre environ 15 cm et environ 30 cm. La longueur du réacteur peut notamment être comprise entre environ 1 cm et environ 100 cm, en particulier entre environ 1 cm et environ 50 cm, plus particulièrement entre environ 10 cm et environ 30 cm.

Selon une modalité particulière, le cordon peut aussi être un prolongement du réacteur, c'est-à-dire venir d'un seul tenant avec lui, éventuellement avec une section moindre. On distingue alors la partie réacteur proprement dite, qui va immobiliser le produit actif, de la partie dite cordon, qui n'est pas destinée à recevoir ce produit actif. Suivant une variante, réacteur, cordon et pièce d'ancrage, viennent d'un seul tenant. Il peut s'agir par exemple d'un ruban. La longueur du réacteur peut alors être typiquement d'environ 10 à environ 60 cm.

Dans le cas de la première modalité, la largeur des faces (du ruban s'il y a lieu) peut être typiquement comprise entre environ 0,5 et environ 3 cm, de préférence entre environ 1 cm et environ 2 cm. L'épaisseur du matériau constituant le réacteur peut être comprise entre environ 0,1 mm et environ 2 mm par exemple de l'ordre de 0,5 mm. La surface d'échange peut notamment varier d'environ 1 cm² à environ 600 cm², pour deux faces. La partie active du réacteur, c'est-à-dire la partie du réacteur qui sera le support du produit actif, peut être limitée à une fraction du réacteur, située à l'opposé du point d'ancrage.

Dans le cas de la deuxième modalité, la surface d'échange du réacteur peut être comprise entre environ 5 cm² et environ 1 800 cm², notamment entre environ 10 cm² et environ 900 cm², de préférence entre environ 100 cm² et environ 300 cm².

A son extrémité opposée à son point d'attache au réacteur, le cordon peut être relié à, ou comporter d'un seul tenant, une pièce d'ancrage. Ce que l'on appelle pièce d'ancrage est un dispositif ou une partie d'un dispositif d'ancrage dans ou sur une paroi tissulaire.

De manière générale, le ruban peut être maintenu en place de plusieurs manières : soit en le fixant à une sonde nasogastrique, soit à une sonde de gastrostomie, soit par un clip endoscopique au niveau de l'antre gastrique, selon la durée prévisible d'utilisation du réacteur ou des pathologies du patient qui justifieraient la pose d'une gastrostomie.

En variante, à la place d'un ruban, on utilise une ou plusieurs pièces centrées sur le cordon, de préférence sur un segment autogyre comme décrit plus haut. La pièce a une forme ou une section essentiellement cylindrique, mais irrégulière afin d'empêcher un flux laminaire au contact de la pièce, cette irrégularité de surface pouvant être obtenue par des variations d'épaisseur ou de diamètre, dans la direction longitudinale et/ou diamétrale de la forme cylindrique, et/ou par la présence de cavités en retrait de la forme cylindrique et/ou de protubérances s'étendant au-delà de la forme cylindrique. A titre d'exemple approprié, on peut citer une forme hélicoïdale ou une forme de segment torse (forme torsadée) par analogie avec les colonnes dites torses, avec des bords extérieurs vifs ou arrondis. Ces pièces s'inscrivent dans un cylindre dont le diamètre peut être compris par exemple entre environ 3 et environ 30 mm, de préférence entre environ 5 et environ 10 mm.

Pour les différents modes de réalisation, le réacteur ou une partie du réacteur (la membrane) peut être formé d'un matériau polymère biocompatible, notamment la membrane semi-perméable ou poreuse, le ruban ou autres pièces peut être réalisé en un matériau polymère naturel ou synthétique résistant à l'environnement dans l'intestin, notamment aux conditions de pH et aux enzymes présentes, notamment les enzymes présentes dans l'environnement intestinal où le réacteur se trouve placé, notamment les enzymes pancréatiques et biliaires, ainsi que les enzymes produites par la flore intestinale, notamment les protéases. En particulier, le polymère peut être du chitosane ou un autre polymère biocompatible, tel que, par exemple : alcool polyvinylique ; poly(méthylméthacrylate) ; copolymère de polyacrylonitrile et de sodium méthanesulfonate, ou PAN-methallylsulfonate, par exemple membrane AN69 (WO 2002-000775) ; copolymères comme le chitosane-polyéthylène glycol ; polymère cellulosique, e.g. acétate de cellulose. De préférence, le polymère, e.g. chitosane, a un degré de réticulation suffisant pour résister à l'environnement dans l'intestin. Il peut aussi être rendu résistant au pH acide ou aux acides par un ou des additifs appropriés. Selon un mode de réalisation, le chitosane est mis en œuvre avec un agent de réticulation, par exemple de la génipine et/ou avec un additif permettant d'améliorer la résistance de la membrane aux acides, par exemple de l'acide caféique.

En variante, le réacteur ou une partie du réacteur (la membrane) peut être formé d'une structure composite, avec un support et un revêtement en matériau polymère synthétique ou naturel biocompatible. Le support peut être notamment un matériau textile, tissé ou non tissé, imprégné et/ou revêtu (coating) avec un matériau polymérique tel que décrit ci-dessus.

En variante, le réacteur ou une partie du réacteur (la membrane) peut être formé d'une structure textile à base de polymère synthétique (par exemple en polyester e.g. Dacron@) ou à base de métal (par exemple alliage nickel-titane).

L'épaisseur du matériau ou de la membrane peut être comprise entre environ 0,1 mm et environ 2 mm par exemple de l'ordre de 1 mm.

Les pores dans le matériau ou la membrane peuvent avoir un diamètre variable, suivant ce qu'ils sont censés devoir laisser passer ou arrêter. De manière générale, le diamètre des pores peut se situer entre 0,1 nm et 500 nm. Entre 0,1 nm et 5 nm, de préférence entre 0,5 nm et 2 nm, les pores laissent passer les substances telles que les sucres et les molécules actives, et retiennent notamment les enzymes et les micro-organismes. On pourra régler le diamètre des pores au moment de la fabrication du matériau, par exemple en modifiant : la concentration du polymère, e.g. chitosane, dans le solvant (e.g. acide acétique pour le chitosane), le ratio chitosane/agent de réticulation, le poids moléculaire du polymère, e.g. chitosane avant dissolution et mise en forme de membrane. On pourra aussi coller à la surface du réacteur des membranes semi-perméables commerciales (par exemple, en acétate de cellulose ou en AN69, ...), dont le degré de porosité peut être choisi dans une large gamme. Dans une variante, ces membranes commerciales pourront être recouvertes d'une couche des polymères décrits dans l'invention (par exemple chitosane modifié par ajout d'agents de réticulation) pour améliorer leur résistance aux conditions physico-chimiques de l'intestin et conserver plus longtemps leurs propriétés de semi-perméabilité.

Dans le cas du réacteur comportant au moins 2 compartiments, la porosité des deux membranes semi-perméables ou poreuses peut être différente.

Dans un mode de réalisation, le matériau ou la membrane comporte des pores qui ne laissent pas passer les protéases. On utilise notamment une telle membrane pour un réacteur creux destiné à renfermer des enzymes qui doivent être protégées des protéases présentes dans le liquide intestinal, sans empêcher le substrat de ces enzymes de pénétrer à l'intérieur du réacteur.

Une membrane poreuse à base de chitosane peut être préparée par un procédé comprenant les étapes suivantes : préparer une solution dans un rapport de 5 à 15 (en mg), de chitosane à 0,75 à 1,25 (en mL) d'acide acétique dilué à 0,4 à 0,6 % en volume dans de l'eau ; agiter pendant 2h ; ajouter un agent de réticulation, par exemple de la génipine, notamment de 0,002 à 0,005 % en masse par volume de génipine ; de l'acide caféique, notamment de 0,001 à 0,005 % en masse par volume d'acide caféique ; agiter pendant 30 min ; verser sur un support anti-adhésif ; sécher lentement, notamment pendant une durée de 2 à 4 jours à température ambiante.

Pour les différents modes de réalisation, le cordon ou la partie cordon a une longueur qui permet, en tenant compte du point d'attache tissulaire, de positionner le réacteur à l'endroit voulu dans l'intestin, notamment dans le duodénum, de préférence en aval de l'abouchement du canal biliaire. Le cordon ou la partie cordon pourra notamment présenter une longueur comprise entre environ 10 cm et environ 20 cm, notamment entre environ 10 cm et environ 15 cm

La pièce d'ancrage du dispositif peut être attachée à la paroi tissulaire (par exemple paroi de l'estomac), par tout moyen de fixation connu de l'homme du métier, par exemple lien, clip ou agrafe. La fixation du cordon à la pièce d'ancrage, lorsque ces pièces ne viennent pas d'un seul tenant, peut être réalisée de toute manière connue, par exemple par collage, soudure, couture, à l'aide de nœuds.

Dans un mode de réalisation particulier, la pièce d'ancrage est un stent pylorique, qui est fixé dans l'antre gastrique, juste en amont du pylore. Il peut notamment s'agir d'un stent dont la structure se déploie pour venir s'immobiliser dans l'antre gastrique en amont du pylore, comme dans le cas du stent du dispositif Endobarrier^{®}.

Selon un autre mode de réalisation, le réacteur comprend au moins une pièce, un bloc ou masse en matériau biocompatible, rattaché au cordon. Dans une modalité particulière, le réacteur comporte un chapelet de plusieurs pièces, par exemple de 2 à 20 pièces, attachées ensemble par le cordon. Avantageusement, chaque pièce est renfermée dans une structure reliée au cordon, par exemple un filet ou analogue, par exemple réalisé en polyester e.g. Dacron@. Ces pièces peuvent être formées par compression de matériau polymère biocompatible éventuellement associé à divers ingrédients favorisant la mise en forme. A titre d'exemple de matériau polymère pour former les pièces, on peut citer le chitosane et l'alginate de baryum. Suivant une modalité particulière, la ou les enzymes d'intérêt sont mélangées au matériau polymère, e.g. chitosane. On peut effectuer ce mélange, puis mettre le mélange sous forme, par exemple par compression. On peut se référer au procédé de fabrication décrit dans la demande de brevet français n°1452534 déposée le 25 mars 2014.

Un matériau similaire peut être utilisé pour former les pièces de matériau mentionnées plus haut, centrées sur le cordon tel que le polyvinyl alcool réticulé et expansé, à l'état amorphe, cristallin ou composite, les matériaux du groupe des polyamines ou des polyesters biocompatibles tels que le polyester de polyvinyl alcool et acide polyacrylique. Ces matériaux polymères pouvant avoir des longueurs de chaîne variables, des structures branchées et réticulées diverses et sous forme de copolymères en formulations différentes conférant au matériau final une structure et une porosité choisie pour la fonction à assurer. La nature hydrophile ou hydrophobe de ce matériau conditionne la liaison durable des protéines enzymatiques, des effecteurs protéiques ou des microbiotes par affinité et adsorption. Des radicaux chimiques peuvent être également utilisés pour renforcer ces liaisons par des liaisons covalentes fortes.

Selon un autre mode de réalisation, le réacteur implantable est un stent intestinal ou digestif qui peut être revêtu en tout ou partie d'un matériau biocompatible, de préférence un matériau polymère biocompatible, ou qui peut comporter une partie formée d'un tel matériau biocompatible, par exemple une tresse ou structure textile, éventuellement revêtue d'une matière polymérique selon l'invention. Ledit matériau peut immobiliser le produit actif ou permettre le développement d'un biofilm. De préférence, le stent est du type tubulaire formé d'un treillis qui se déploie pour épouser l'intérieur de l'intestin. Ce stent peut être dimensionné pour une pose dans le duodénum, l'intestin grêle ou le colon.

Le matériau biocompatible est de préférence un polymère tel que décrit à propos du mode de réalisation précédent. En particulier, le polymère peut être du chitosane ou autre polymère biocompatible par exemple : alcool polyvinylique, poly(méthylméthacrylate), ou copolymères comme le chitosane-polyéthylène glycol ou un mélange de ces polymères. De préférence, le polymère, e.g. chitosane, a un degré de réticulation suffisant pour résister à l'environnement dans l'intestin. Selon un mode de réalisation, le chitosane est mis en œuvre avec par exemple de la génipine et de l'acide caféique.

Il peut aussi s'agir d'une structure textile, notamment tresse, en polymère synthétique (par exemple en polyester e.g. Dacron@) ou à base de métal (par exemple alliage nickel-titane), notamment revêtue d'un polymère tel que décrit supra.

Par définition, dans une forme de réalisation, le dispositif de l'invention peut comprendre le réacteur, le cordon ou partie de cordon et la pièce d'ancrage.

L'invention a non seulement pour objet le réacteur brut tel qu'il vient d'être décrit, mais aussi le réacteur comportant le produit actif. Le produit actif est à l'intérieur du volume intérieur du réacteur, avec ou sans matériau de remplissage ou matrice, et/ou à sa surface, selon le type de réacteur.

Le produit actif peut notamment être choisi parmi les enzymes, des micro-organismes tels que bactéries ou levures, ou selon un exemple non couvert par les revendications : des amas cellulaires eucaryotes, et en particulier des bactéries capables de digérer des oligosaccharides.

Les enzymes peuvent notamment être utilisées avec un réacteur comportant un volume intérieur dans lequel l'enzyme peut être contenue, en présence ou non d'un matériau de remplissage ou autre matrice, ou mélangées à un matériau tel que le chitosane pour former une structure comme il a été décrit précédemment. Comme enzymes, on peut notamment utiliser des disaccharidases et/ou des enzymes qui transforment les sucres « simples », notamment ceux issus de l'action des disaccharidases sur les disaccharides. De préférence on associe les deux types d'enzymes. Parmi les disaccharidases, on peut notamment citer la lactase, la maltase et la bêta-fructosidase, l'alpha-glucosidase, la bêta-glucosidase, la bêta-galactosidase, seules ou en mélanges. Comme enzymes transformant les sucres simples, on peut notamment citer, seules ou en mélange, la glucose oxydase et la glucose déshydrogénase, éventuellement associée à l'aldose réductase.

La consommation du glucose avant absorption intestinale est un mode de réalisation très intéressant. On prévoit de préférence des disaccharidases, car les glucides après l'intestin se présentent préférentiellement sous forme de disaccharides, et une enzyme permettant de transformer les sucres simples issus de l'action de l'enzyme précédente. La glucose oxydase transforme le glucose en gluconate et constitue une première modalité. Celui-ci sera moins bien absorbé par l'intestin que le glucose, car les membranes intestinales ne disposent pas de transporteurs spécifiques du gluconate, alors que plusieurs protéines transmembranaires sont dédiées au transport du glucose. Par ailleurs, une fois absorbé par l'organisme, une grande majorité du gluconate est excrétée directement par le rein. Des enzymes telles que la glucose déshydrogénase, qui ont besoin d'un cofacteur, constitue une deuxième modalité, car il faut prévoir la régénération du cofacteur. Par exemple, la glucose déshydrogénase produit aussi du gluconate, mais elle utilise le NAD+ (*nicotinamide adénine dinucléotide*) ou le NADP+ comme accepteur d'électrons, et non l'oxygène. Dans ce cas, pour régénérer le NADP+, on peut prévoir la présence d'aldose réductase, capable de réduire le glucose en sorbitol. Or, le sorbitol n'est que peu absorbé par l'intestin, il est d'ailleurs utilisé comme traitement de l'obésité (sans effet secondaire notable à moins de 17g/kg/24h), car il favorise le transit intestinal. Compte tenu du fait que les 10 L environ de chyme qui sortent quotidiennement de l'estomac contiennent environ 20 g.L-1 de glucides, pour éliminer en 24 h environ 200 mmol de glucose (soit environ 38 g ou environ 8% de la ration calorique), on prévoit une activité enzymatique d'environ 140 UI. Ainsi, le réacteur de l'invention pourra comprendre de l'aldose réductase, notamment en association avec la glucose déshydrogénase.

Dans un mode de réalisation particulier, le réacteur comprend au moins 2 compartiments. Dans le premier se trouve une première enzyme, par exemple une disaccharidase et éventuellement un cofacteur, dans l'autre une deuxième enzyme, par exemple une enzyme transformant les sucres simples, et éventuellement un cofacteur. Les seuils de coupure des membranes sont adaptés pour éviter les fuites des enzymes et cofacteurs que les compartiments contiennent tout en permettant aux substrats de passer. Ainsi, un premier compartiment contient une ou plusieurs disaccharidases (e.g. lactase, maltase, beta-fructosidase, alpha-glucosidase, beta-glucosidase, beta-galactosidase) et la membrane a un seuil de coupure (par exemple environ 500 ou 1000 g/mole) permettant aux disaccharides de passer dans ce compartiment, où ils sont transformés en sucres simples, notamment glucose; le deuxième compartiment contient du NAD⁺ ou NADP⁺ et une ou plusieurs enzymes transformant les sucres simples (e.g. glucose oxydase et/ou glucose déshydrogénase), il est séparé du premier par une membrane ayant un seuil de coupure (par exemple environ 300 g/mole) permettant de retenir son contenu et de laisser passer les sucres simples qui se sont formés dans le premier compartiment.

Selon l'invention, le réacteur peut comprendre des enzymes telles que la lactase ou d'autres galactosidases, susceptibles de traiter l'intolérance au lactose, ou des glutenases, notamment la cystéine endoprotéase ou les enzymes ALV001, ALV002 et ALV003, décrites dans les demandes de brevet WO 2005-107786, WO 2008-115428, WO 2008-115411, WO 2010-021752, WO 2010-42203, pour traiter l'intolérance au gluten.

Les enzymes et les micro-organismes peuvent aussi jouer un rôle dans le développement orienté du microbiote. Par exemple, l'action de la glucose oxydase présente l'intérêt de consommer de l'oxygène et de produire des protons. Combinée à la catalase (qui dismute le H₂O₂ produit et évite l'action potentiellement néfaste de H₂O₂ sur la paroi intestinale), la glucose oxydase nécessite 1 mmol d'O₂ pour oxyder 2 mmol de glucose et permet de consommer de l'oxygène. Le prélèvement d'une quantité significative de l'oxygène exercera une forte pression de sélection sur le microbiote, en faveur des bactéries anaérobies. De même, la production de protons va favoriser les bactéries acidophiles. Ainsi, le réacteur de l'invention pourra comprendre de la catalase, notamment en association avec la glucose oxydase.

Des protons peuvent être produits, par exemple lors de la transformation de glucose en gluconate, ou par l'action de certaines bactéries (*Lactobacillus acidophilus*, notamment) sur les nutriments présents dans la lumière intestinale. Des ions ammonium peuvent être produits, par exemple par action de l'uréase sur l'urée. Ainsi, le réacteur de l'invention pourra comprendre de l'uréase ou toute autre enzyme utile pour influer sur le microbiote ou le liquide intestinal, éventuellement en association avec la glucose oxydase et/ou la glucose déshydrogénase. Les réactions chimiques peuvent donc être orientées pour modifier localement le pH du tube digestif. Ces modifications vont modifier la composition du microbiote. Or, le microbiote interagit avec de nombreux organes et systèmes physiologiques. Il est soupçonné d'être impliqué dans des pathologies aussi diverses que l'obésité, le diabète, l'hypercholestérolémie, certains cancers, la maladie d'Alzheimer, etc. Si l'on couple l'intervention sur le microbiote via la modification de paramètres comme le pH et l'observation de paramètres cliniques ou biologiques, on pourra donc obtenir l'effet thérapeutique souhaité pour un patient donné.

Le réacteur peut permettre la production in situ d'inhibiteurs d'enzymes digestives et/ou des molécules, comme le diméthyl-butanol, qui réduit le TMA-oxydé, ou bien des hormones notamment stimulant la satiété. Pour ce faire le réacteur pourra contenir des bactéries naturelles sélectionnées pour les fonctions désirées, ou des bactéries génétiquement modifiées pour réaliser ces fonctions de production de molécules d'intérêt.

Les bactéries pourront être soit placées dans le volume intérieur du réacteur, soit appliquées à sa surface, selon la configuration retenue pour le réacteur.

Comme bactéries, on peut citer notamment celles utilisées comme probiotiques, dont les espèces appartenant aux genres *Bacillus, Lactobacillus, Bifidobacterium, Streptococcus* (qui produisent une fermentation lactique), *Escherichia ou encore Pseudomonas.*

Comme levures, on peut citer les espèces du genre *Saccharomyces,* notamment des levures du genre *Saccharomyces* modifiée génétiquement pour bloquer la synthèse d'éthanol.

On peut utiliser des micro-organismes naturels ou génétiquement modifiés, par exemple pour consommer des glucides, des lipides ou des protéines en produisant des molécules moins absorbables par l'intestin ou influant sur le microbiote en modifiant les conditions physico-chimiques.

Certains médicaments ou hormones (insuline, Glucagon-Like-Peptide 1 (GLP1), L-Dopa par exemple) ne peuvent être que difficilement ingérés, car il est délicat de les protéger du pH gastrique et des enzymes intestinales. Or, ces molécules peuvent être produites par des bactéries ou levures modifiées par génie génétique, ou dans certains cas par des cellules eucaryotes (cellules de Langerhans animales ou humaines pour la production d'insuline, par exemple). L'encapsulation de tels micro-organismes dans le réacteur proposé permet de synthétiser en permanence ces molécules dans des zones immédiatement capables de permettre leur absorption par l'intestin. Ceci présente un intérêt comme nouvelle approche thérapeutique dans des pathologies telles que le diabète de type I ou de type II, ou la maladie de Parkinson.

Le réacteur peut comprendre des micro-organismes ou, selon un exemple non couvert par les revendications, des cellules eucaryotes capables de produire au moins une molécule d'intérêt thérapeutique, notamment un polypeptide par exemple L-dopa, GLP1 ou insuline, notamment des micro-organismes génétiquement modifiés pour produire au moins un polypeptide, par exemple L-dopa, GLP1 ou insuline.

On peut aussi utiliser des micro-organismes, e.g. bactéries ou levures, génétiquement modifiées pour produire des molécules capables d'inhiber ou d'inactiver des molécules impliquées dans certaines pathologies (anti-vitamines K, anticorps anti-peptide tau précurseur des protéines qui s'accumulent dans la maladie d'Alzheimer, etc.).

Les microorganismes peuvent être utilisés pour produire un ou des acides aminés essentiels. Les besoins nutritionnels en acides aminés essentiels sont importants en santé humaine. A titre d'exemple, une carence en tryptophane est connue pour engendrer des syndromes dépressifs (voie de la sérotonine) ou d'insomnie (melatonine). On peut utiliser des micro-organismes pour produire du tryptophane, ou autres dérivés de la voie de la sérotonine.

Dans certaines situations physiopathologiques, certains acides aminés non essentiels peuvent le devenir. A titre d'exemple, chez les phenylcétonuriques, l'apport en tyrosine est essentiel. On peut utiliser des micro-organismes pour produire la tyrosine.

On peut utiliser des microroganismes qui produisent un ou plusieurs de ces acides aminés essentiels.

Dans le cadre de la lutte contre la pénurie alimentaire on peut également envisager de conférer au réacteur la propriété de dégrader la cellulose afin d'apporter une source supplémentaire en glucides, naturellement non assimilables par l'homme. En particulier, le réacteur pourra contenir des bactéries qui sécrètent des enzymes capables de réaliser la dégradation de la cellulose.

On peut également utiliser des micro-organismes, notamment des bactéries génétiquement modifiées, pour produire des molécules capables de diminuer certaines réactions intestinales inflammatoires, notamment l'élafine, impliquée dans l'intolérance au gluten.

A titre d'exemple de bactérie utilisable pour produire une molécule d'intérêt, par modification génétique (notamment par insertion d'une cassette ou d'un système d'expression de ladite molécule), on peut citer *Lactobacillus salivarius*, ou encore *Pseudomonas sp.,* notamment *P. aeruginosa*, de préférence sous forme atténuée. On peut également citer les bactéries *E. coli* acceptables, telles que la souche Nissle 1997. L'expression de la molécule d'intérêt peut être sous le contrôle de l'expression du gène codant pour cette molécule, par exemple via une molécule signal ingérable par le patient.

On peut aussi utiliser des bactéries produisant de l'insuline ou d'autres molécules du type acetohexamide ou acarbose. L'acarbose est un pseudotétrasaccharide d'origine microbienne. Au niveau de la bordure en brosse de l'intestin, l'acarbose agit par inhibition compétitive des alpha-glucosidases. Il diminue ainsi la dégradation des carbohydrates (dioligo- et polysaccharides) en monosaccharides absorbables. L'acarbose diminue donc l'hyperglycémie postprandiale, sans entraîner d'hyperinsulinémie ni de modification de poids.

On peut aussi utiliser des bactéries produisant la molécule en réponse à la présence d'un inducteur contenu dans le bol alimentaire (type analogue du lactose) ou autre, par exemple un promoteur inductible à l'aspirine, ce qui confère une propriété de contrôle de l'activité du réacteur.

Le réacteur peut notamment être à usage unique. L'élément de fixation peut aussi être à usage unique ou peut être réutilisable, ce qui peut être le cas lorsque l'on prévoit de retirer le réacteur lorsque celui-ci a atteint sa date limite d'efficacité ou d'utilisation, et le remplacer par un réacteur neuf ou un autre type de réacteur. Le remplacement peut se faire à partir de l'élément de fixation ou plus aisément à partir d'un point intermédiaire, par exemple sur l'émerillon ou autre dispositif séparant le cordon d'ancrage et le cordon porteur du réacteur.

Le réacteur selon l'invention est utilisé pour apporter et rendre biodisponible un produit actif à l'intérieur de l'intestin, notamment duodénum, grêle ou colon. Cette utilisation vise notamment à générer, grâce à ce produit actif, une réaction chimique avec une ou des molécules présentes (cas notamment du réacteur comprenant une ou des enzymes) ou à produire (cas notamment du réacteur comprenant un ou des micro-organismes) une ou des molécules qui vont avoir une fonction chimique ou biologique ou encore à modifier le microbiote.

Une utilisation peut être notamment la consommation de glucose présent dans l'intestin, en particulier dans le duodénum et/ou le jéjunum, avant qu'il ne soit absorbé. Le réacteur peut alors comprendre une ou des enzymes du type disaccharidases (e.g. maltase, lactase, beta-fructosidase, alpha-glucosidase, beta-glucosidase, beta-galactosidase) et de la glucose oxydase et/ou glucose déshydrogénase, ou similaire.

Dans cette utilisation, on peut aussi utiliser un réacteur comprenant un micro-organisme capable de consommer les glucides, lipides ou protéines, associé des enzymes susceptibles de dégrader aussi ces molécules, telles que glucose oxydase et/ou glucose déshydrogénase, un ou une association de micro-organismes pour consommer les disaccharides et monosaccharides (notamment glucose), ou encore des disaccharidases associées à un micro-organisme capable de consommer les sucres simples, notamment le glucose.

Dans cette utilisation, le réacteur peut aussi comprendre de la catalase et/ou de l'uréase.

D'autres utilisations potentielles sont la transformation de protéines, de lipides, ou de molécules comme l'oxygène, l'alcool, le lactose, le gluten. Dans ce cas, le réacteur comprend des enzymes, notamment la lactase ou d'autres galactosidases, la cystéine endoprotéase ou les enzymes ALV001, ALV002 et ALV003.

D'autres utilisations potentielles sont la production de molécules biologiquement intéressantes, notamment de molécules qui sont sensibles au passage dans le tractus digestif (aux enzymes digestives ou au pH notamment), par exemple L-dopa, GLP1, insuline, ou des molécules capables de diminuer certaines réactions intestinales inflammatoires, notamment l'élafine, impliquée dans l'intolérance au gluten.

D'autres utilisations potentielles sont d'utiliser le réacteur comme une base d'ensemencement ou de modification du microbiote, en apportant des micro-organismes, notamment bactéries, et/ou des molécules qui vont influer sur le microbiote, par exemple en produisant ou en consommant protons, ion ammonium, oxygène.

La description a aussi pour objet, non couvert par les revendications, la méthode de traitement d'un mammifère, homme ou animal, de préférence homme, dans laquelle on implante un réacteur selon l'invention, et on l'y maintient sur une durée suffisante, par exemple plusieurs jours, semaines, mois ou années. Le réacteur est positionné dans la lumière intestinale, comme il a été indiqué supra en fonction du mode de réalisation. La méthode peut notamment prévoir la fixation d'un dispositif d'ancrage dans la paroi de l'estomac et le rattachement du réacteur à ce dispositif, ou la mise en place d'un stent dans l'antre gastrique auquel est raccordé ou auquel l'on raccorde le réacteur, ou encore la mise en place d'un stent intestinal. Le réacteur peut donc prendre l'une des formes mentionnées supra. Ce réacteur, une fois mis en place, permet une réaction chimique avec une ou des molécules présentes (cas notamment du réacteur comprenant une ou des enzymes) ou la production (cas notamment du réacteur comprenant un ou des micro-organismes) d'une ou de molécules qui vont avoir une fonction chimique ou biologique ou encore de micro-organismes.

La description a ainsi pour objet, non couvert par les revendications, une méthode de traitement d'un mammifère, homme ou animal, de préférence homme, dans laquelle on implante dans son système digestif un dispositif selon l'une quelconque des revendications précédentes, comprenant un réacteur portant un élément actif, et un élément de fixation du réacteur à une paroi intestinale ou gastrique, la méthode comprenant la fixation de l'élément de fixation à la paroi intestinale ou gastrique, et le positionnement du réacteur dans la lumière intestinale, ce grâce à quoi l'élément actif produit ou peut produire son effet biologique ou chimique.

Parmi les applications, liées notamment au métabolisme des sucres, des lipides ou des protéines, notamment du glucose, on peut citer notamment le traitement et/ou la prévention de l'obésité, du diabète de type I ou II, de l'hypercholestérolémie. Cette méthode peut donc utiliser un réacteur comprenant les associations d'enzymes et/ou de micro-organismes qui ont étés présentées supra.

La méthode de l'invention peut être utilisée dans d'autres applications, comme le traitement d'intolérances alimentaires, maladie d'Alzheimer, maladie de Parkinson, diabète de type I, etc.

La description peut donc recouvrir, selon des aspects non couverts par les revendications, des méthodes thérapeutiques et des méthodes non thérapeutiques, par exemple des méthodes de traitement probiotique, de traitement de confort ou de traitement de type complément alimentaire.

L'invention va être maintenant décrite plus en détail à l'aide de modes de réalisation pris à titre d'exemples non limitatifs et se référant au dessein dans lequel :
- La figure 1 est une représentation schématique d'un mode de réalisation d'un dispositif « segmenté ».
- La figure 2 montre le dispositif de la figure 1 en coupe transversale au niveau d'un segment.
- La figure 3 est une représentation schématique d'un mode de réalisation d'un dispositif segmenté selon un autre mode de réalisation.
- La figure 4 est une représentation schématique d'un segment du dispositif de la figure 3.

### Exemple 1 :

On réalise une dissolution de 200 mg de chitosane dans 20 mL d'acide acétique dilué à 0,5 % en volume dans de l'eau. On ajoute au mélange initial un agent de réticulation, de la génipine à 0,0045 % en masse par volume (g/100 mL) et de l'acide caféique dans une proportion de 0,0032 % en masse par volume (g/100 mL) dans la solution visqueuse de chitosane après 2 h d'agitation. La génipine est préalablement solubilisée dans une solution de 12 % de diméthylsulfoxyde (DMSO) et 88 % d'eau (H₂O). L'acide caféique est préalablement solubilisé à 4 % dans de l'éthanol.

Après 30 min d'agitation, on a prélève 3 g de ce mélange qu'on a étale sur un support lisse non adhésif (diamètre 28 cm), par exemple une coupelle de polystyrène antistatique, et on sèche pendant une durée de 2 à 4 jours à température ambiante (une température comprise entre 20 à 30 °C est appropriée). Dans un autre essai, on sèche pendant trois jours à 25 °C.

On obtient ainsi des membranes nanoporeuses flexibles. Des expériences effectuées par la demanderesse ont montré que cette flexibilité était liée au fait que le séchage est effectué pendant une longue durée à température ambiante. Cette caractéristique n'est pas obtenue par exemple si l'on utilise des températures de séchage supérieures à 40° C. Pour une épaisseur de film de l'ordre de 7 à 15 µm, par exemple 10 µm, on a obtenu une membrane poreuse avec des diamètres moyens de pores de l'ordre de 1 à 10 nanomètres. On préférera se placer dans des conditions où ce diamètre moyen est de l'ordre de 5 à 8 nm pour permettre de laisser passer le glucose et de filtrer les composés de plus grandes dimensions.

### Exemple 2 :

Dans un tube cylindrique (d'environ 1 cm de diamètre et d'environ 20 cm de longueur) d'acétate de cellulose présentant un seuil de coupure à 5 000 g.mole⁻¹ fermé à une extrémité, on introduit des bactéries génétiquement modifiées pour produire de l'insuline jusqu'à une hauteur de 4 cm dans le tube. On ferme l'autre extrémité du tube.

Puis, on aplatit le tube comprenant les bactéries et on place ce tube aplati (épaisseur environ 1 à 2 mm) sur un premier ruban en Dacron@ (environ 20 cm de longueur et environ 1,5 cm de largeur) puis on dépose un deuxième ruban en Dacron@ (environ 20 cm de longueur et environ 1,5 cm de largeur) pour former un dispositif selon l'invention qui a la forme d'un sandwich en ayant cousu ensemble les bords des deux rubans.

On fixe l'ensemble à un clip gastrique au moyen d'un ruban en Dacron@ (environ 15 cm de longueur et environ 1 cm de largeur). Le clip est positionné par voie endoscopique dans la paroi de l'antre gastrique. À travers le canal opérateur de l'endoscope et avec une pince, on déroule le ruban et on le fait passer au travers du pylore. Le réacteur contenant les micro-organismes est alors positionné dans le duodénum, après l'ampoule de Vater où arrivent les canaux pancréatique et biliaire.

### Exemple 3 :

On utilise un ruban tissé en Dacron@ pour former un ruban de 50 cm x 1 cm. A l'une de ses extrémités, ce ruban est suturé à un clip gastrique. Le dispositif est mis à incuber pendant 48 h dans un milieu de culture de bactéries du type *Lactobacillus acidophilus.* Le clip est positionné par voie endoscopique dans la paroi de l'antre gastrique. A travers le canal opérateur de l'endoscope et avec une pince on déroule le ruban et on le fait passer au travers du pylore.

### Exemple 4 :

À un clip gastrique, on coud un ruban de Dacron@ de 50 cm x 1 cm. On ajoute aux 200 mg de chitosane de l'exemple 1 un mélange de 60 mg de glucose oxydase (100 Unités Internationales/mg), de 60 mg de catalase (1 000 Unités Internationales/mg) et de 60 mg de beta-galactosidase (100 Unités Internationales/mg). Après 30 min d'agitation, on étale 3 g du mélange sur une des faces du ruban, sur une longueur de 35 cm à partir de l'extrémité libre du ruban tissé en Dacron^{®} de 50 cm x 1 cm. On laisse sécher pendant 3 jours à 25°C. Le clip est positionné par voie endoscopique dans la paroi de l'antre gastrique. À travers le canal opérateur de l'endoscope et avec une pince, on déroule le ruban et on le fait passer au travers du pylore. Le réacteur contenant les micro-organismes est alors positionné dans le duodénum, après l'ampoule de Vater où arrivent les canaux pancréatique et biliaire.

### Exemple 5 :

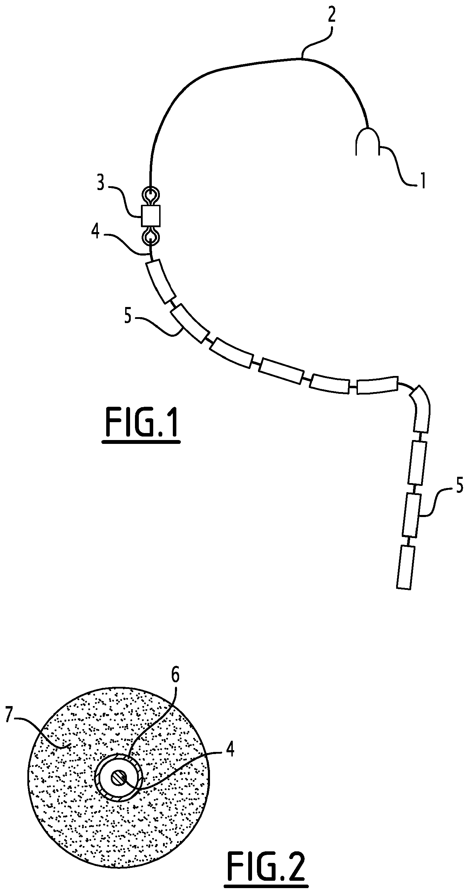

Le dispositif de la Figure 1 est formé d'une agrafe stomacale 1 fixée à une extrémité d'un filin ou cordon d'ancrage 2, lequel est fixé à son autre extrémité à une extrémité d'un émerillon 3. Le filin d'ancrage est un fil de téflon monobrin d'une longueur d'environ 200 mm et de diamètre de 1,0 mm. Un deuxième cordon ou filin 4 de téflon monobrin a une longueur d'environ 300 mm et un diamètre de 1,0 mm. Il est fixé à l'autre extrémité de l'émerillon 3, de telle manière que les filins 2 et 4 et l'émerillon peuvent tourner les uns par rapport aux autres. Sur le filin 4 sont montés, sur toute sa longueur, dix segments 5, dits segments autogyres car ils peuvent tourner autour du filin 4. On comprendra mieux la structure en se référant à la figure 2 qui montre, en coupe transversale, un segment 5 et le filin 4 qu'il entoure. Chaque segment est formé d'un tube 6 de téflon de diamètre extérieur de 3 mm, de diamètre interne de 1,6 mm et de longueur de 30 mm. Sur chaque tube 6 est fixé une couche épaisse de PVA expansé 7, le diamètre extérieur du segment ainsi obtenu étant de 6 mm. Le PVA expansé peut être le support d'un élément actif, par exemple de développement d'un biofilm. L'ensemble des segments peut ainsi être traité par l'élément actif, ou seulement certains d'entre eux, selon la zone de l'intestin dans laquelle on veut agir. Le montage par segments librement rotatifs permet d'éviter que le filin 4 ne vrille sous l'influence des forces qui s'exerceront sur le dispositif à l'intérieur de l'intestin. La longueur du filin 4 va dépendre de la zone la plus éloignée devant être atteinte dans l'intestin. Une longueur de 150 mm peut être utilisée pour la zone duodénale. Une longueur supplémentaire de 150 à 350 mm peut être utilisée pour la zone jéjunale. De son côté, l'agrafe stomacale 1 est apte à être fixée à la paroi intérieure de l'estomac et son point d'ancrage ainsi que la longueur du filin d'ancrage 2 permettent à ce dernier de s'étendre jusqu'au voisinage du pylore.

Dans un autre mode de réalisation, le dispositif diffère de celui qui vient d'être décrit par l'absence de l'émerillon 3, par un filin d'ancrage tubulaire unique (2 + 4) de diamètre extérieur 2 mm, diamètre intérieur 1 mm et longueur 500 mm, dix segments 5 de longueur de 30 mm, de diamètre intérieur de 2,2 mm. Dans ce mode de réalisation, le filin d'ancrage tubulaire permet l'utilisation d'un fil guide rigide coaxial pour la mise en place du dispositif dans le tube digestif. Pour cela, le fil guide rigide est mis en place au préalable depuis l'ouverture buccale, puis le dispositif est enfilé sur ce fil guide et poussé jusqu'à la distance la plus éloignée devant être atteinte dans l'intestin.

### Exemple 6 :

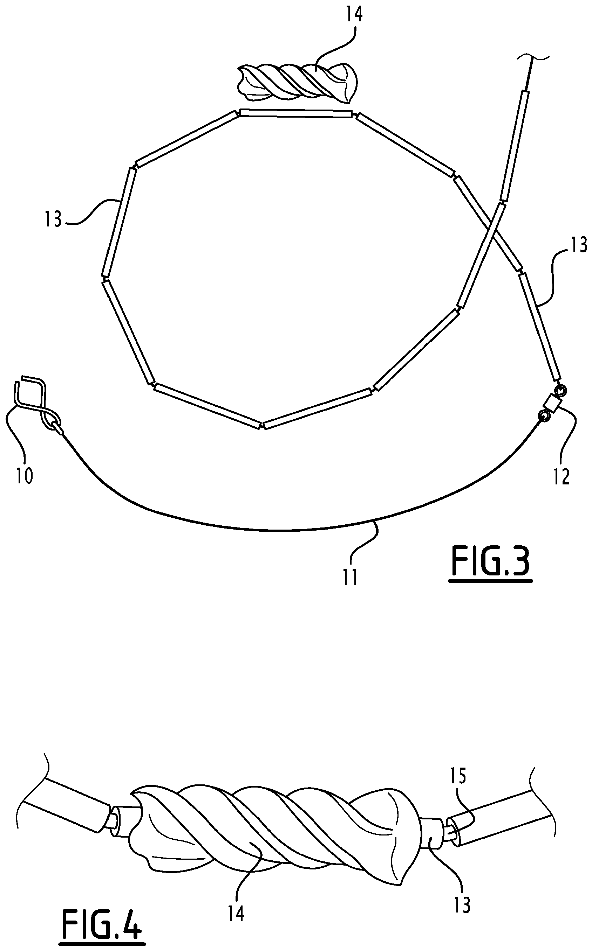

Ce mode de réalisation, représenté aux figures 3 et 4, est assez proche de celui de la figure 1 en ce qui concerne sa structure générale, avec, dans l'ordre, une agrafe stomacale 10, un filin monobrin d'ancrage 11, un émerillon 12, des segments 13 montés rotatifs sur un filin monobrin de nylon 15. Le repère numérique 14 désigne une structure en spirale qui vient se fixer sur l'un des segments 13. Comme à l'exemple 5, la structure en spirale, par exemple en PVA expansé, peut être le support d'un élément actif, par exemple de développement d'un biofilm. Un ou plusieurs des segments peut/peuvent être équipé(s) d'un tel support, et l'on peut aisément régler la longueur du filin 15 et la longueur et le nombre de segments, pour placer le ou les supports à l'endroit voulu dans l'intestin.

## Revendications

1. Dispositif implantable dans la cavité intestinale, comprenant un réacteur et un élément de fixation du réacteur comprenant une pièce d'ancrage configurée pour se fixer dans ou sur une paroi tissulaire, l'élément de fixation permettant de maintenir le réacteur en place dans la cavité intestinale, le réacteur est configuré pour renfermer ou supporter un produit actif, sous forme d'au moins un parmi une enzyme et un microorganisme, le microorganisme étant choisi parmi une bactérie ou une levure.

2. Dispositif selon la revendication 1, dans lequel le réacteur comprend une membrane semi-perméable ou un revêtement semi-perméable ou un matériau semi-perméable, ayant notamment la forme d'un ruban, d'un tube ou d'une structure comprenant plus de deux faces.

3. Dispositif selon la revendication 1 ou 2, dans lequel le réacteur est fixé à un cordon qui le solidarise à l'élément de fixation.

4. Dispositif selon la revendication 3, dans lequel le cordon est entouré par des segments cylindriques autogyres dont le diamètre intérieur est supérieur au diamètre du cordon, de sorte que les segments sont libres en rotation autour du cordon pour éviter au cordon de se vriller sous l'action de contraintes mécaniques s'exerçant sur le dispositif.

5. Dispositif selon la revendication 3 ou 4, dans lequel le cordon est monté sur un émerillon.

6. Dispositif selon l'une des revendications précédentes, dans lequel le dispositif de fixation est choisi parmi un clip gastrique, un stent pylorique et un stent intestinal.

7. Dispositif selon l'une des revendications précédentes, dans lequel le réacteur comporte une membrane semi-perméable ou poreuse, la membrane semi-perméable ou poreuse délimitant un volume intérieur fermé et formant une surface extérieure du dispositif.

8. Dispositif selon l'une des revendications précédentes, dans lequel le réacteur comprend un matériau biocompatible particulaire, granulaire ou une masse en matériau biocompatible.

9. Dispositif selon l'une des revendications précédentes, dans lequel le réacteur
• contient une disaccharidase, de préférence maltase, lactase, beta-fructosidase, alpha-glucosidase, beta-glucosidase, beta-galactosidase, seule ou combinée à une enzyme capable de dégrader au moins un monosaccharide, tel le glucose, notamment choisie parmi la glucose oxydase et une combinaison de glucose déshydrogénase, d'aldose réductase et de NADP ou de NAD ; ou
• contient des bactéries capables de dégrader le glucose, notamment des bactéries de type *Lactobacillus,* en particulier des bactéries de type *Lactobacillus acidophilus* ; ou
• contient des enzymes capables de dégrader le gluten, notamment des glutenases, en particulier les enzymes ALV001, ALV002 et ALV003,
• un inhibiteur d'enzyme digestive et/ou une hormone coupe-faim,
• des microorganismes produisant un ou des acides aminés essentiels,
• des microorganismes dégradant la cellulose,
• du microorganismes produisant du diméthyl-butanol,
• des bactéries produisant de l'insuline, de l'acetohexamide ou de l'acarbose.

10. Dispositif selon l'une des revendications précédentes, comprenant des micro-organismes génétiquement modifiés, par exemple *Pseudomonas aeruginosa* ou *E. coli,* ou des cellules eucaryotes, capables de produire au moins une molécule d'intérêt thérapeutique, notamment un polypeptide, par exemple L-dopa, GLP1, insuline ou élafine.

11. Dispositif selon la revendication précédente, dans lequel la production de la molécule d'intérêt thérapeutique, est inductible par une molécule signal ingérée par l'hôte.

12. Dispositif selon l'une des revendications précédentes, dans lequel l'au moins un parmi une enzyme et un microorganisme est propre à modifier les conditions physicochimiques dans la cavité intestinale de façon à modifier le microbiote intestinal.

13. Dispositif selon l'une des revendications précédentes, pour son utilisation pour générer au moins une réaction chimique avec une ou des molécules présentes dans l'intestin ou pour produire au moins une molécule biologiquement active, notamment pour la production de molécules d'intérêt thérapeutique ou pour la consommation de glucose présent dans l'intestin, en particulier dans le duodénum et/ou le jéjunum, avant qu'il ne soit absorbé, le réacteur comprenant :
• une ou des enzymes du type disaccharidase (de préférence maltase, lactase, beta-fructosidase, alpha-glucosidase, beta-glucosidase, beta-galactosidase) et de la glucose oxydase et/ou une combinaison de glucose déshydrogénase, d'aldose réductase et de NAD ou de NADP ; ou
• des micro-organismes, notamment des micro-organismes génétiquement modifiés, capables de produire au moins une molécule d'intérêt thérapeutique, notamment un polypeptide, par exemple L-dopa, GLP1, insuline ou élafine.

14. Dispositif selon l'une des revendications 1 à 6, dans lequel le réacteur comporte au moins deux faces à la surface d'au moins une desquelles un biofilm de micro-organismes est formé, le biofilm permettant un ensemencement du microbiote intestinal.

15. Dispositif selon l'une des revendications 3 à 5 dans lequel le réacteur comporte une ou plusieurs pièces s'inscrivant dans un cylindre et centrées sur le cordon, pièce ayant une forme ou une section essentiellement cylindrique, mais irrégulière afin d'empêcher un flux laminaire au contact de la pièce.

16. Dispositif selon la revendication 15, **caractérisé en ce que** la surface extérieure de la pièce présente une forme hélicoïdale ou une forme de segment torse.

17. Dispositif selon l'une des revendications précédentes, dans lequel la longueur du dispositif est comprise entre environ 5 cm et environ 150 cm.

## Patentansprüche

1. Vorrichtung, die in den intestinalen Hohlraum implantierbar ist, umfassend einen Reaktor und ein Befestigungselement des Reaktors, umfassend ein Verankerungsteil, konfiguriert, um in oder auf einer Gewebewand befestigt zu werden, wobei das Befestigungselement erlaubt, den Reaktor in dem intestinalen Hohlraum an Ort und Stelle zu halten, wobei der Reaktor konfiguriert ist, um einen Wirkstoff in Form von mindestens einem unter einem Enzym und einem Mikroorganismus zu umschließen oder zu tragen, wobei der Mikroorganismus ausgewählt ist aus einer Bakterie oder einer Hefe.

2. Vorrichtung nach Anspruch 1, wobei der Reaktor eine halbdurchlässige Membran oder eine halbdurchlässige Beschichtung oder ein halbdurchlässiges Material umfasst, die/das insbesondere die Form eines Bandes, eines Rohrs oder einer Struktur aufweist, die mehr als zwei Flächen umfasst.

3. Vorrichtung nach Anspruch 1 oder 2, wobei der Reaktor an einer Schnur befestigt ist, die ihn mit dem Befestigungselement verbindet.

4. Vorrichtung nach Anspruch 3, wobei die Schnur von zylindrischen Schraubsegmenten, von denen der Innendurchmesser größer als der Schnurdurchmesser ist, derart umgeben ist, dass die Segmente um die Schnur herum frei drehbar sind, um zu verhindern, dass sich die Schnur unter der Einwirkung von mechanischen Einschränkungen, die auf die Vorrichtung ausgeübt werden, verdreht.

5. Vorrichtung nach Anspruch 3 oder 4, wobei die Schnur an einem Drehgelenk montiert ist.

6. Vorrichtung nach einem der vorstehenden Ansprüche, wobei die Befestigungsvorrichtung ausgewählt ist aus einer Magenklammer, einem Pylorusstent und einem Intestinalstent.

7. Vorrichtung nach einem der vorstehenden Ansprüche, wobei der Reaktor eine halbdurchlässige oder poröse Membran beinhaltet, wobei die halbdurchlässige oder poröse Membran ein geschlossenes Innenvolumen umgrenzt und eine Außenoberfläche der Vorrichtung bildet.

8. Vorrichtung nach einem der vorstehenden Ansprüche, wobei der Reaktor ein granulöses teilchenförmiges biokompatibles Material oder eine biokompatible Materialmasse umfasst.

9. Verfahren gemäß einem der vorstehenden Ansprüche, wobei der Reaktor
• eine Disaccharidase enthält, vorzugsweise Maltase, Lactase, Beta-Fructosidase, Alpha-Glucosidase, Beta-Glucosidase, Beta-Galactosidase, allein oder kombiniert mit einem Enzym, das in der Lage ist, mindestens ein Monosaccharid wie Glucose abzubauen, insbesondere ausgewählt unter Glucoseoxydase und einer Kombination von Glucosedehydrogenase, Aldosereduktase und NADP oder NAD; oder
• Bakterien enthält, die in der Lage sind, Glucose abzubauen, insbesondere Bakterien der Art Lactobacillus, besonders Bakterien der Art Lactobacillus acidophilus, oder
• Enzyme enthält, die in der Lage sind, Gluten abzubauen, insbesondere Glutenasen, besonders die Enzyme ALV001, ALV002 und ALV003,
• einen Verdauungsenzym-Inhibitor und/oder ein Appetitzüglerhormon,
• Mikroorganismen, die eine oder mehrere essentielle Aminosäuren produzieren,
• Mikroorganismen, die Cellulose abbauen,
• Mikroorganismen, die Dimethylbutanol produzieren,
• Bakterien, die Insulin, Acetohexamid oder Acarbose produzieren.

10. Vorrichtung nach einem der vorstehenden Ansprüche, umfassend genetisch modifizierte Mikroorganismen, zum Beispiel Pseudomonas aeruginosa oder E. coli, oder eukaryotische Zellen, die in der Lage sind, mindesten ein Molekül von therapeutischem Interesse zu produzieren, insbesondere ein Polypeptid, zum Beispiel L-Dopa, GLP1, Insulin oder Elafin.

11. Vorrichtung nach vorstehendem Anspruch, wobei die Produktion des Moleküls von therapeutischem Interesse durch ein von dem Wirt eingenommenes Signalmolekül induzierbar ist.

12. Vorrichtung nach einem der vorstehenden Ansprüche, wobei das mindestens eine aus einem Enzym und einem Mikroorganismus geeignet ist, die physikochemischen Bedingungen in dem intestinalen Hohlraum auf eine Weise zu modifizieren, um die Darmflora zu modifizieren.

13. Vorrichtung nach einem der vorstehenden Ansprüche, für seine Verwendung zum Erzeugen mindestens einer chemischen Reaktion mit einem oder mehreren im Darm vorhandenen Molekülen, oder um mindestens ein biologisch aktives Molekül zu produzieren, insbesondere für die Produktion von Molekülen von therapeutischem Interesse oder zum Verbrauch von in dem Darm vorhandener Glucose, besonders in dem Duodenum und/oder dem Jejunum, bevor sie absorbiert wird, wobei der Reaktor umfasst:
• ein oder mehrere Enzyme der Disaccharidase-Art (vorzugsweise Maltase, Lactase, Beta-Fructosidase, Alpha-Glucosidase, Beta-Glucosidase, Beta-Galactosidase) und Glucoseoxydase und/oder eine Kombination von Glucosedehydrogenase, Aldosereduktase und NAD oder NADP; oder
• Mikroorganismen, insbesondere genetisch modifizierte Mikroorganismen, die in der Lage sind, mindestens ein Molekül von therapeutischem Interesse zu produzieren, insbesondere ein Polypeptid, zum Beispiel L-Dopa, GLP1, Insulin oder Elafin.

14. Vorrichtung nach einem der Ansprüche 1 bis 6, wobei der Reaktor mindestens zwei Flächen auf der Oberfläche beinhaltet, wobei auf mindestens einer von denen ein Biofilm von Mikroorganismen gebildet ist, wobei der Biofilm eine Beimpfung der Darmflora erlaubt.

15. Vorrichtung nach einem der Ansprüche 3 bis 5, wobei der Reaktor eine oder mehrere Stücke beinhaltet, die in einen Zylinder passen und an der Schnur zentriert sind, wobei das Stück eine Form oder eine Sektion aufweist, die im Wesentlichen zylindrisch, aber unregelmäßig ist, um einen Laminarfluss in Kontakt mit dem Stück zu vermeiden.

16. Vorrichtung nach Anspruch 15, **dadurch gekennzeichnet, dass** die Außenoberfläche des Stücks eine Spiralform oder eine Torsosegmentform vorweist.

17. Vorrichtung nach einem der vorstehenden Ansprüche, wobei die Länge der Vorrichtung zwischen etwa 5 cm und etwa 150 cm liegt.

## Claims

1. A device implantable into the intestinal cavity, comprising a reactor and an element for fastening the reactor comprising an anchor configured to be fastened in or on a tissue wall, the fastening element allowing holding the reactor in place in the intestinal cavity, the reactor is configured to enclose or support an active product, in the form of at least one of an enzyme and a microorganism, the microorganism being selected from a bacterium or a yeast.

2. The device according to claim 1, wherein the reactor comprises a semi-permeable membrane or a semi-permeable coating or a semi-permeable material, having in particular the form of a ribbon, a tube or a structure comprising more than two faces.

3. The device according to claim 1 or 2, wherein the reactor is fastened to a cord which secures it to the fastening element.

4. The device according to claim 3, wherein the cord is surrounded by self-rotating cylindrical segments whose inner diameter is greater than the diameter of the cord, such that the segments are free to rotate around the cord to prevent the cord from twisting under the action of mechanical stresses being exerted on the device.

5. The device according to claim 3 or 4, wherein the cord is mounted on a swivel.

6. The device according to one of the preceding claims, wherein the fastening device is selected from a gastric clip, a pyloric stent and an intestinal stent.

7. The device according to one of the preceding claims, wherein the reactor includes a semi-permeable or porous membrane, the semi-permeable or porous membrane defining a closed internal volume and forming an outer surface of the device.

8. The device according to one of the preceding claims, wherein the reactor comprises a particulate, granular biocompatible material or a mass made of biocompatible material.

9. The device according to one of the preceding claims, wherein the reactor
• contains a disaccharidase, preferably maltase, lactase, beta-fructosidase, alpha-glucosidase, beta-glucosidase, beta-galactosidase, alone or combined with an enzyme capable of degrading at least one monosaccharide, such as glucose, in particular selected from glucose oxidase and a combination of glucose dehydrogenase, aldose reductase and NADP or NAD; or
• contains bacteria capable of degrading glucose, in particular bacteria of the *Lactobacillus* type, in particular bacteria of the *Lactobacillus acidophilus* type, or
• contains enzymes capable of degrading gluten, in particular glutenases, in particular the enzymes ALV001, ALV002 and ALV003,
• a digestive enzyme inhibitor and/or an appetite suppressant hormone,
• microorganisms producing one or more essential amino acids,
• cellulose-degrading microorganisms,
• microorganisms producing dimethyl-butanol,
• bacteria producing insulin, acetohexamide or acarbose.

10. The device according to one of the preceding claims, comprising genetically modified microorganisms, for example *Pseudomonas aeruginosa* or *E. coli,* or eukaryotic cells, capable of producing at least one molecule of therapeutic interest, in particular a polypeptide, for example L-dopa, GLP1, insulin or elafin.

11. The device according to the preceding claim, wherein the production of the molecule of therapeutic interest is inducible by a signal molecule ingested by the host.

12. The device according to one of the preceding claims, wherein the at least one of an enzyme and a microorganism is suitable for modifying the physicochemical conditions in the intestinal cavity so as to modify the intestinal microbiota.

13. The device according to one of the preceding claims, for its use to generate at least one chemical reaction with one or more molecules present in the intestine or to produce at least one biologically active molecule, in particular for the production of molecules of therapeutic interest or for the consumption of glucose present in the intestine, in particular in the duodenum and/or the jejunum, before it is absorbed, the reactor comprising:
• one or more enzymes of the disaccharidase type (preferably maltase, lactase, beta-fructosidase, alpha-glucosidase, beta-glucosidase, beta-galactosidase) and glucose oxidase and/or a combination of glucose dehydrogenase, aldose reductase and NAD or NADP; or
• microorganisms, in particular genetically modified microorganisms, capable of producing at least one molecule of therapeutic interest, in particular a polypeptide, for example L-dopa, GLP1, insulin or elafin.

14. The device according to one of claims 1 to 6, wherein the reactor includes at least two faces on the surface of at least one of which a biofilm of microorganisms is formed, the biofilm allowing a seeding of the intestinal microbiota.

15. The device according to one of claims 3 to 5 wherein the reactor includes one or more parts inscribed in a cylinder and centred on the cord, a part having a shape or a section which is essentially cylindrical, but irregular in order to prevent a laminar flow in contact with the part.

16. The device according to claim 15, **characterised in that** the outer surface of the part has a helical shape or a twisted segment shape.

17. The device according to one of the preceding claims, wherein the length of the device is between about 5 cm and about 150 cm.
